(19) Europäisches Patentamt
European Patent Office
Office européen des brevets

(11) Publication number: **0 037 134 A1**

(12) **EUROPEAN PATENT APPLICATION**

(21) Application number: 81200263.2

(22) Date of filing: 09.03.81

(51) Int. Cl.$^3$: C 07 D 237/24
A 01 N 43/58

(30) Priority: 26.03.80 GB 8010194

(43) Date of publication of application: 07.10.81 Bulletin 81/40

(84) Designated Contracting States: CH DE FR GB IT LI NL

(71) Applicant: SHELL INTERNATIONALE RESEARCH MAATSCHAPPIJ B.V.
Carel van Bylandtlaan 30
NL-2596 HR DEN HAAG(NL)

(72) Inventor: Haddock, Ernest
187 Queensborough Road
Sheerness Sheppey Kent(GB)

(74) Representative: Keuzenkamp, Abraham et al.
P.O. Box 302
NL-2501 CH Den Haag(NL)

(54) **Pyridazine derivatives, process for their preparation, compositions containing them and methods of regulating the growth of plants, increasing the yield of soya bean plants and sterilizing the male anthers of plants, including small grain cereal plants, using them as well as a method of producing F1 hybrid seed.**

(57) Compounds of general formula

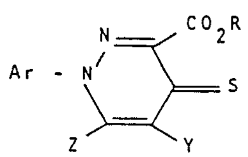

(1)

in which Ar represents an optionally substituted phenyl group; Y represents a hydrogen or halogen atom or an alkyl group; Z represents an alkyl group; and R represents an hydrogen atom, one equivalent of a cation, or an alkyl group; have useful plant growth regulating properties.

EP 0 037 134 A1

Pyridazine derivatives, process for their preparation, compositions containing them and methods of regulating the growth of plants, increasing the yield of soya bean plants and sterilizing the male anthers of plants, including small grain cereal plants, using them as well as a method of producing $F_1$ hybrid seed

This invention relates to pyridazine derivatives, process for their preparation, compositions containing them and a method of regulating the growth of plants using them.

In the past, the vast majority of agricultural chemical research work was directed towards the development of chemical fertilizers and the development of chemicals to combat pests, diseases and weeds which reduce the yield of crops. The belief was that by adequately feeding the plant and by optimizing its environment, the yields of the crop could be maximized.

In recent years it has become clear that it is possible to affect the growth of plants in a much more fundamental way, that is by the application of chemicals which affect the biochemical processes occuring in the plant. Much research is now being directed towards the development of chemical plant growth regulants which are capable of altering the biochemistry of plants in such a way that useful effects are achieved. One important field of research is into the stimulation of crops to produce higher yields. It would for example be most useful to be able to increase the yield of soyabeans, a valuable source of protein, by means of a chemical treatment.

The invention provides a compound of the general formula

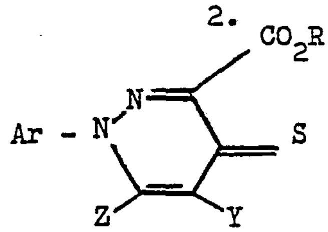

(I)

in which Ar represents an optionally substituted phenyl group; Y represents a hydrogen or halogen atom or an alkyl group; Z represents an alkyl group; and R represents a hydrogen atom, one equivalent of a cation, or an alkyl group.

Preferably Y represents a hydrogen or chlorine atom or an alkyl group having up to 6, preferably up to 4, carbon atoms. More preferably Y represents a hydrogen atom.

Preferably Z represents an alkyl group having up to 6, especially up to 4, carbon atoms. More preferably Z represents a methyl group.

Preferably Ar represents a phenyl group which is unsubstituted or substituted by one or more of the same or different substituents selected from halogen atoms and alkyl, alkoxy, alkylthio, cyano, aryl and carboxy groups. More preferably, Ar represents a phenyl group which is unsubstituted or substituted by one or more halogen atoms and/or methyl groups. An especially prefered group Ar is a monohalophenyl group, especially a 4-chlorophenyl group.

A cation represented by R may for example be an alkali metal ion, one equivalent of an alkaline earth metal ion, or an ammonium ion optionally substituted by up to 4 alkyl groups each preferably having up to 4 carbon atoms.

An alkyl group represented by R preferably has up to 6, especially up to 4, carbon atoms.

Preferably R represents a hydrogen atom, an alkali metal, especially a sodium, ion, or an alkyl group having up to 4 carbon atoms, especially a methyl, ethyl or isopropyl group.

The invention also provides a process for the preparation of a compound of the general formula I, which comprises reacting a compound of the general formula

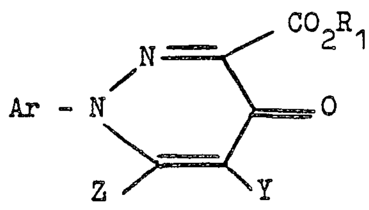

(II)

in which Ar, Y and Z have the meanings given for the general formula I, and $R_1$ represents an alkyl group, with a thiolating agent; and in the event of a compound of the general formula I being required in which R represents a hydrogen atom or a cation, hydrolysing the resulting ester to produce the required compound.

An especially suitable thiolating agent is phosphorus pentasulphide.

The reaction is suitably performed in the presence of an inert solvent, for example an ether such as dioxan or tetrahydrofuran. The reaction temperature may vary over a wide range, for example from 10 to 150°C, preferably 10 to 100°C. It may be convenient to carry out the reaction at room temperature or at the reflux temperature of the solvent used.

A resulting ester may be converted into the corresponding free acid or a salt thereof by the hydrolysis methods normally used for such reactions. Hydrolysis under acid or neutral conditions will produce the free acid, whereas hydrolysis under alkaline conditions will produce a salt.

The compounds according to the invention exhibit useful plant growth regulating effects, and the invention therefore provides a plant growth regulating composition which comprises a compound of the general formula I together with a suitable carrier. The invention also provides a method of regulating the growth of a plant, which comprises treating the plant, the seed from which it is to be grown, or the medium in which it is growing or to be grown, with a compound or a composition according to the invention.

Different plant growth regulating effects will be obtained depending on the growth stage of the plant when treated. One preferred embodiment of the invention is a method of increasing

the yield of soyabeans, which comprises treating a soyabean plant or the soil in which it is growing with a compound or a composition according to the invention, the treatment being carried out at a growth stage after the flowers of the plant have differentiated. Generally the treatment may be carried out from the time at which the flowers of the plant have differentiated, and throughout the time when the flowers are open.

A second preferred embodiment of the invention is a method of sterilizing the male anthers of a plant, which comprises treating the plant or the soil in which it is growing with a compound or a composition according to the invention, the treatment being carried out at a growth stage such that sterilization occurs.

This second embodient of the invention makes it possible to sterilize the male anthers of plants, especially small-grain cereal plants, without substantially affecting female fertility. The resulting male-sterile plant may then be cross-pollinated by pollen from a plant of a different strain, producing $F_1$ hybrid seed. This process is especially useful for self-pollinating plants. Thus the invention also provides a method of producing $F_1$ hybrid seed which comprises cross-pollinating a plant which has been treated by the sterilizing process of the present invention with a second plant of a different strain.

The appropriate growth stage for carrying out the sterilizing process will vary from species to species, but generally it is appropriate to treat the plant at a growth stage before or during the earlier stages of flower differentation. For small-grain cereal plants, such as wheat or barley, the treatment is most suitably carried out when the plant is at a stage of growth between late tillering and emergence of the ear.

Preferably the growth of plants is regulated by the application of the active compound at a dosage in the range of from 0.05 to 2 kg/ha, preferably 0.25 to 1 kg/ha.

A carrier in a composition according to the invention is any material with which the active ingredient is formulated to facilitate application to the locus to be treated, which may for example

be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids;

the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 and 75% w of active ingredient and usually contain, in addition to solid inert carrier, 3-10% w of a dispersing agent and, where necessary, 0-10% w of stabiliser(s) and/or other additives such as penetrants or stickers. Dusts are usually formulated as a dust concentrate having a similar compositions to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-25% w active ingredient and 0-10% w of additives such as stabilisers, slow release modifiers and binding agents. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a

stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The compositions of the invention may also contain other ingredients, for example, other compounds possessing plant growth regulating, insecticidal or fungicidal properties.

The following Examples illustrate the invention.

Example 1 Preparation of 1-(4-chlorophenyl)-3-ethoxycarbonyl-6-methylpyridazin-4-thione.

1-(4-chlorophenyl)-3-ethoxycarbonyl-6-methylpyridazin-4-one (5.0 g) and phosphorus pentasulphide (10.0 g) were added to dry dioxan (100 ml) and stirred at room temperature for 48 hours. The liquid was then decanted off from the solid, and evaporated down to give a deep red oil. This was purified by chromotography over silica gel using diethyl ether as eluant. The desired product was obtained as a deep orange solid, melting point 157-158°C.

Elemental Analysis

| | C | H | N |
|---|---|---|---|
| Calculated for $C_{14}H_{13}ClN_2O_2S$ | 54.5 | 4.2 | 9.1 |
| Found | 53.7 | 4.3 | 8.7 |

Example 2 Demonstration of soyabean growth regulation

The plants used in the evaluation of the compound of Example 1 were determinate soyabean cultivars (e.g. Fiskeby V).

Liquid formulations of the compound were applied to plants at an early stage of flower development. The formulations used consisted of 60% water, 40% acetone which contained 0.4% TRITON X155 (Trade Mark), and an amount of the test compound to give a spray application eqivalent to 1.0 Kg/ha.

In order to induce nodulation, the soyabean seeds were inoculated with a commercial strain of Rhizobium ("Nitrogerm", Root Nodule Pty., Ltd., Australia) prior to sowing in a loam: grit (5:1) mixture in 5" diameter pots. Plants were maintained at 21-25°C under 14 hr. day length and watered by sub-irrigation. Treatments were as foliar applications to three plants, applied in a volume equivalent to 632 litres per hectare. After treatment the plants were set out in a randomised block design. After four weeks the total pod number per plant was recorded. Results were derandomised and mean values calculated and expressed as a percentage of the untreated controls.

It was found that the number of pods on the plants treated with the compound of Example 1 was 153% of the number of pods on the control plants.

Example 3 Demonstration of pollen-suppressing activity

Spring wheat, variety Sicco, was propagated in a glass-house in 8 cm pots containing a loam-based compost. Supplementary lighting was provided by high-pressure mercury vapour lamps to give a constant day length of 16 hours. The temperature was maintained at approximately 20°C.

The compound of Example 1 was formulated as an aqueous solution containing 0.1% nonidet P 40 (trade mark) as wetting agent and 1% acetone to aid solubility. This formulation was diluted with water to a concentration of 1000 ppm and sprayed onto plants in a volume equivalent to 650 litres/ha. The plants were treated at the growth stage when the second node of the plant was just detectable.

At ear emergence but before anthesis, 3 heads from each main stem and 2 heads from the tillers in each pot were placed in cellophane bags to prevent cross-pollination. At maturity, the

bagged ears were harvested, and seed set was recorded and compared with untreated controls.

It was found that the seed set in the treated plants was reduced by 54% compared with the untreated controls, clearly illustrating the ability of the compound of Example 1 to sterilize the male anthers of the wheat.

CLAIMS

1. A compound of general formula

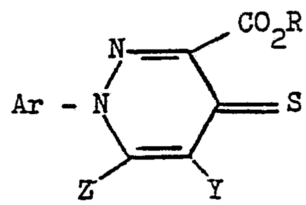

(I)

in which Ar represents an optionally substituted phenyl group; Y represents a hydrogen or halogen atom or an alkyl group; Z represents an alkyl group; and R represents an hydrogen atom, one equivalent of a cation, or an alkyl group.

2. A compound as claimed in claim 1, in which Y represents a hydrogen atom.

3. A compound as claimed in either claim 1 or claim 2, in which Z represents a methyl group.

4. A compound as claimed in any one of claims 1 to 3, in which Ar represents a phenyl group which is unsubstituted or substituted by one or more of the same or different substituents selected from halogen atoms and alkyl, alkoxy, alkylthio, cyano, aryl and carboxy groups.

5. A compound as claimed in claim 4, in which Ar represents a monohalophenyl group.

6. A compound as claimed in any one of claims 1 to 5 in which R represents a hydrogen atom, an alkali metal ion, one equivalent of an alkaline earth metal ion, an ammonium ion optionally substituted by up to 4 alkyl groups each having up to 4 carbon atoms, or an alkyl group having upto 6 carbon atoms.

7. A compound as claimed in claim 6, in which R represents a hydrogen atom, an alkali metal ion or an alkyl group having up to 4 carbon atoms.

8. A process for the preparation of a compound as claimed in claim 1, which comprises reacting a compound of the general formula

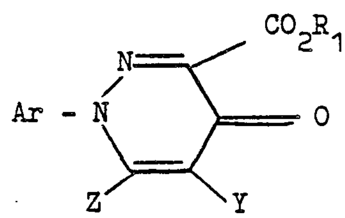

(II)

in which Ar, Y and Z have the meanings given in claim 1, and $R_1$ represents an alkyl group, with a thiolating agent; and in the event of a compound of the general formula I being required in which R represents a hydrogen atom or a cation, hydrolysing the resulting ester to produce the required compound.

9. A process as claimed in claim 8, in which the thiolating agent is phosphorus pentasulphide.

10. A compound as claimed in claim 1, whenever prepared by a process as claimed in any one of claims 8 to 9.

11. A plant growth regulating composition which comprises a compound as claimed in any one of claims 1 to 7 and 10 together with a carrier.

12. A composition as claimed in claim 11, which comprises at least two carriers, at least one of which is a surface-active agent.

13. A method of regulating the growth of a plant, which comprises treating the plant, the seed from which it is to be grown, or the medium in which it is growing or is to be grown, with a compound as claimed in any one of claims 1 to 7 and 10 or a composition as claimed in either claim 11 or claim 12.

14. A method as claimed in claim 13, which comprises increasing the yield of soya beans by treating a soya bean plant or the soil in which it is growing with a compound as claimed in any one of claims 1 to 7 and 10 or a composition as claimed in either claim 11 or claim 12, at a growth stage after the flowers of the plant have differentiated.
15. A method as claimed in claim 13, which comprises sterilizing the male anthers of a plant by treating the plant or the soil in which it is growing with a compound as claimed in any one of claims 1 to 7 and 10 or a composition as claimed in either claim 11 or claim 12, at a growth stage such that sterilization occurs.
16. A method as claimed in claim 15, in which the plant is a small-grain cereal plant.
17. A method of producing $F_1$ hybrid seed which comprises cross-pollinating a plant which has been treated by a process as claimed in either claim 15 or claim 16, with a second plant of a different strain.

0037134

European Patent Office

# EUROPEAN SEARCH REPORT

Application number
EP 81 20 0263

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | DE - A - 2 808 975 (ROHM & HAAS)<br>* Pages 1-28 *<br>-- | 1,11-17 | C 07 D 237/24<br>A 01 N 43/58 |
| | HELVETICA CHIMICA ACTA, vol. 39, 1956, pages 1741-1754<br>Basel, CH<br>A. STAEHELIN et al. "Heilmittelchemische Studien in der Heterocyclischen Reihe. Pyridazine VIII"<br>* Page 1748, 1749 table 1; 1750, table 2 *<br>-- | 1 | TECHNICAL FIELDS SEARCHED (Int. Cl.³)<br>C 07 D 237/24<br>A 01 N 43/58 |
| X | US - A - 3 562 272 (H. OTT)<br>* Column 1-4, lines 3-8 *<br>----- | 8-10 | |

CATEGORY OF CITED DOCUMENTS
X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

The present search report has been drawn up for all claims

| Place of search | Date of completion of the search | Examiner |
|---|---|---|
| The Hague | 09-07-1981 | FRANCOIS |